# EUROPEAN PATENT SPECIFICATION

(11) **EP 2 190 494 B1**
(45) Date of publication and mention of the grant of the patent: **18.10.2017**
(21) Application number: 08826967.5
(22) Date of filing: 04.08.2008
(51) Int. Cl.: A61L 31/10, A61L 31/16

(54) **IMPLANTABLE MEDICAL DEVICES HAVING THIN ABSORBABLE COATINGS**
IMPLANTIERBARE MEDIZINISCHE VORRICHTUNGEN MIT DÜNNEN ABSORPTIONSFÄHIGEN BESCHICHTUNGEN
DISPOSITIFS MÉDICAUX IMPLANTABLES COMPORTANT DE MINCES REVÊTEMENTS RÉSORBABLES

(30) Priority: 08.08.2007 US 891131
(43) Date of publication of application: 02.06.2010
(73) Proprietor: Abbott Cardiovascular Systems Inc., Santa Clara, CA 95054-2807 (US)
(72) Inventor: KLEINER, Lothar Walter, Los Altos, CA 94023-0087 (US); DESNOYER, Jessica, Renee, San Jose, CA 95129 (US); MASLANKA, Bozena, Sofia, Aptos, CA 95003 (US); TROLLSAS, Mikael, O., San Jose, CA 95124 (US); HOSSAINY, Syed, F.A., Hayward, CA 94544 (US); TANG, Yiwen, Sunnyvale, CA 94086 (US); GLAUSER, Thierry, Redwood City, CA 94062 (US); NGO, Michael, H., San Jose, CA 95128 (US)
(74) Representative: ZBM Patents - Zea, Barlocci & Markvardsen
(86) International application number: PCT/US2008/072152
(87) International publication number: WO 2009/020936

(56) References cited:
- US-A1- 2005 112 171
- US-A1- 2005 288 481
- US-A1- 2007 026 041

## Description

### FIELD OF THE INVENTION

The invention relates to implantable medical devices. In particular, the invention relates to coatings useful for the controlled delivery of bioactive agents from implantable medical devices such as stents.

### BACKGROUND

One commonly applied technique for the local delivery of drugs is through the use of drug eluting stents. Stents are scaffoldings, usually cylindrical or tubular in shape, functioning to physically hold open, and if desired, to expand the wall of the passageway. Typically, stents are capable of being compressed for insertion through small cavities via small catheters, and then expanded to a larger diameter once at the desired location. Drug delivery can be accomplished by depositing or forming a polymer coating on the surface of the stent. Various deposition techniques are well known in the art. One Example includes spray application of polymer dissolved in a solvent and a drug added thereto. One example of a polymer that has been explored is polyester amide (PEA).

A bioabsorbable PEA polymer that has been explored in drug delivery stents is the co-poly-{[N,N'-sebacoyl-bis-(L-leucine)-1,6-hexylene diester]-[N,N'-sebacoyl-L-lysine 4-amino-TEMPO amide]} (PEA-TEMPO)) polymer. One of its shortcomings is that its permeability to bioactive agents such as everolimus is too high, necessitating thicker coatings (e.g., approximately 13 to 14 microns) to slow the drug release profile of the agent through the coating. The term "thickness" can refer to the distance between opposite surfaces of a polymeric matrix that is used in the fabrication of a medical device or coating. The thickness can refer to that of a single layer or a combination of layers. In general, for a given polymer, thicker coatings require longer degradation times, which can be undesirable.

Other bioabsorbable polymers, such as D,L polylactic acid ("D,L-PLA"), have a slower permeability to bioactive agents such as everolimus, thereby allowing for thinner coatings. However, D,L-PLA may not impart adequate mechanical integrity to a stent. Thus, it is difficult to obtain a desired bioactive agent release profile from coatings, while maintaining the overall stability and mechanical strength required of a stent.

The goal in drug delivery stents is to achieve desirable release profiles while maintaining optimal coating characteristics with regard to permeability, stability and mechanical strength. Target release profiles should be achieved while the coating retains the physical characteristics to withstand the forces that are applied during deployment, crimping and expansion of the stent. The art continues to develop more reliable ways to control the release of a bioactive agent from coatings, while maintaining the overall stability and mechanical strength required of a stent. Such control can be important to obtain the desired therapeutic effects or reduce any adverse physiological effects that may otherwise occur from the stent therapy. In addition to providing a way to improve the bioactive, bio-beneficial, and/or diagnostic results currently obtained from the administration of agents, control over the release rate of agents can assist in maintaining the physical and mechanical properties of stent coatings. Accordingly, control over the release of agents is an important design consideration and one of the next hallmarks in the development of stent technology.

The patent application US 2005/112171 discloses a stent having a coating, the coating comprising a first biologically erodable polymer, that is poly(caprolactone), having a glass transition temperature below -50°C. The coating additionally includes a biologically erodable polymeric additive mixed with the first polymer, wherein the additive is a polymer having a glass transition temperature of -50°C or greater. The additive is selected from a group consisting of poly(L-lactide), poly(D,L-lactide), poly(L-lactide-co-D,L-lactide), poly(glycolide), poly(glycolide-co-L-lactide), poly(glycolide-co-D,L-lactide), and other polymers and mixtures thereof. The coated stent is used to release everolimus.

### SUMMARY OF THE INVENTION

Described herein is a coating on an implantable device as disclosed in claim 1. The matrix layer or the release profile controlling layer includes a matrix phase and a dispersed phase, where the dispersed phase is substantially or completely immiscible with the matrix phase. The
dispersed phase and the matrix phase is substantially compatible such that the dispersed phase would not substantially cause the formation of channels or interconnected voids or pores in the matrix phase. In some embodiments, the bioactive agent is substantially impermeable through the dispersed phase. In some embodiments, the bioactive agent is less permeable through the dispersed phase than the matrix phase. In some embodiments, the term "substantially immiscible" can refer to about less than about 10% of the minor dispersed phase being miscible with the major matrix phase.

The coating described herein allows for controlled release of the bioactive agent from the coating and can have various degradation rates. In some embodiments, the coating is capable of complete degradation in a period of less than about 24 months, about 12 months, or about 6 months in a physiological environment.

The bioactive agent is everolimus.

A medical device having a coating described herein can be used to treat, prevent, or ameliorate a medical condition such as atherosclerosis, thrombosis, restenosis, hemorrhage, vascular dissection or perforation, vascular aneurysm, vulnerable plaque, chronic total occlusion, claudication, anastomotic proliferation (for vein and artificial grafts), bile duct obstruction, urethra obstruction, tumor obstruction, and combinations thereof.

### BRIEF DESCRIPTION OF THE DRAWINGS

Figure 1 illustrates a scanning electron microscope (SEM) image of a portion of a stent having a PEA-TEMPO/D,L-PLA blend as a release profile controlling layer, where PEA-TEMPO is the matrix phase polymer and D,L-PLA is the dispersed phase polymer.
Figure 2 illustrates a SEM image of a portion of a stent having a reservoir layer of everolimus and a PEA-TEMPO/D,L-PLGA blend, as well as a second layer of a PEA-TEMPO and D,L-PLA blend for use as a release profile controlling layer.
Figure 3 is a graph of the cumulative percent of everolimus released into porcine serum (one day and three days) from stents having various coatings of PEA-TEMPO and D,L-PLA blends, as compared to a stent having an everolimus/poly(vinylidene fluoride-co-hexafluoropropene) coating.

### DETAILED DESCRIPTION

Described herein is a coating on an implantable device that comprises a matrix layer and/or a release profile controlling layer comprising a bioactive agent as disclosed in claim 1. The matrix layer or the release profile controlling layer includes a matrix phase and a dispersed phase, where the dispersed phase is substantially or completely immiscible with the matrix phase. In some embodiments, the bioactive agent is substantially impermeable through the dispersed phase. In some embodiments, the bioactive agent is less permeable through the dispersed phase than the matrix phase. In some embodiments, the term "substantially immiscible" can refer to about less than about 10% of the minor dispersed phase being miscible with the major matrix phase.

The coating described herein allows for controlled release of the bioactive agent from the coating and can have various degradation rate. In some embodiments, the coating is capable of complete degradation in a period of less than about 24 months, about 12 months, or about 6 months in a physiological environment.

The bioactive agent is everolimus.

A medical device having a coating described herein can be used to treat, prevent, or ameliorate a medical condition such as atherosclerosis, thrombosis, restenosis, hemorrhage, vascular dissection or perforation, vascular aneurysm, vulnerable plaque, chronic total occlusion, claudication, anastomotic proliferation (for vein and artificial grafts), bile duct obstruction, urethra obstruction, tumor obstruction, and combinations thereof

As used herein, the term "dispersed phase" refers to a precipitate phase in a matrix layer or release profile controlling layer while the term "matrix phase" refers to a substantially continuous phase in the matrix layer or release profile controlling layer. The matrix phase is generally amorphous or more amorphous than the "dispersed phase." The dispersed phase can be less permeable to a bioactive agent than a matrix phase. Generally, the dispersed phase is the minor phase while the matrix phase is the major phase.

The term "crystallinity" refers to regions in which polymer chains align with one another, usually parallel, to form crystalline lattices in an effort to obtain the most favorable thermodynamics. "Crystallinity" can be measured by the weight percent (wt%) of the crystalline portion of the polymer.

The term "bioactive agent" refers any substance that can be used for therapeutic, prophylactic, or diagnostic purposes. A therapeutic purpose refers to the treatment of an ongoing disease or disorder, the goal being to cure it or at least ameliorate its symptoms. A prophylactic purpose refers to the administration of a bioactive agent before any disease or disorder has manifested itself or the administration after the disease or disorder has been subjected to therapeutic treatment to prevent recurrence of the disease or disorder or of symptoms of the disease or disorder.

The term "release profile" refers to the amount of bioactive agent released from a coating as a function of time.

The term "burst" refers to elution of about 80% to about 99.5% of a bioactive agent from a coating on an implantable medical device released within about 1 to about 3 days following implantation.

The term "reservoir layer" refers to a bioactive agent-containing polymer layer.

The term "release profile controlling layer" refers to a layer disposed on the reservoir layer, which modifies the release profile of the bioactive agent from the device.

The terms "disposed over" means that the coating is placed essentially uniformly over the target region of the surface of the implantable medical device. While "applied to" has generally the same meaning as "disposed over," the latter infers that there may be something else, such as, without limitation, another layer of material, between the coating layers or between a reservoir layer and the surface of the implantable medical device whereas the former infers that the coating is placed directly onto the other reservoir layer or onto the surface of the implantable medical device.

As used herein, the term "implantable device" is used interchangeably with the term "medical device" or "implantable medical device."

In some embodiments, the dispersed phase can be less than 50%, 45%, 40%, 30%, 25%, 20%, 15%, 10%, or 5% by volume in the coating. In some embodiments, the dispersed phase should constitute less than 50% but greater than 1%, 5%, 10%, 20%, 25% or 30% of the coating. In other embodiments, the dispersed phase can be between 49% to 10%, 40% to 10%, 40% to 20%, 40% to 30%, 30% to 10%, or 30% to 20%. A morphology having a dispersed phase and a matrix phase allows for slowed drug release kinetics of a bioactive agent because the bioactive agent is less permeable or impermeable through the dispersed phase than through the matrix phase. Thus, thicker coatings for use in slowing the release profile of a drug are not necessary to slow the drug release, and thinner coatings, for example, less than about 5 microns, may be used to obtain optimal drug release profiles. In one preferred embodiment, the coating can be less than about 10 microns, about 5 microns, about 4 microns, about 3 microns, about 2 microns, or about 1 micron.

Without being limited by any particular theory, it is believed that by including an immiscible dispersed phase in a matrix phase polymer, the release rate of the bioactive agent through the construct is slowed because the bioactive agent must circumvent the dispersed phase, forcing a longer diffusion path of the bioactive agent through the construct. Because the agent's permeability through the coating is hindered by the dispersed phase polymer within the matrix phase polymer, the agent's permeability through the coating is slowed, enabling a more controlled release of an agent through a coating. In this way, the coating need not be made thicker in efforts to slow the release rate of the bioactive agent from the coating.

The coatings herein exhibit a beneficial balance of a desired release profile, permeability, loading, mechanical strength and stability. That is, the coatings disclosed herein are capable of various release profiles of a bioactive agent, while maintaining the integrity of the coating, physical stability, and mechanical strength.

The coatings can be used on balloon expandable or self-expanding stents. The stent may be used in any part of the vasculature, including neurological, carotid, coronary, renal, aortic, iliac, femoral, or other peripheral vasculature. There is no inherent limitation on the length, diameter, strut pattern, or strut thickness.

In some embodiments, the multi-phase coating can be formed by a blend, mixture, or combination of two or more polymers. For example, in one embodiment, a matrix phase polymer is blended, mixed, or combined with a dispersed phase polymer to form a coating including a matrix phase comprising the matrix phase polymer and a dispersed phase comprising the dispersed phase polymer, where the dispersed polymer is substantially or completely immiscible in the matrix phase polymer, causing phase separation of the matrix phase and the dispersed phase. It is also contemplated that the multi-phase coating can be formed by a single polymer having immiscible polymeric blocks, such as to cause phase separation.

In some embodiments, the matrix phase polymer is amorphous or less crystalline than the dispersed phase polymer. In some embodiments, the matrix phase polymer has a glass transition temperature of -20°C to 100°C, more narrowly -10°C to 50°C, and even more narrowly -10°C to 30°C.

The matrix phase polymer comprises PEA-TEMPO.

The dispersed phase of the coatings is substantially or completely immiscible in the matrix phase and is less permeable to the bioactive agent than the matrix phase. In one embodiment, the dispersed phase is substantially or completely impermeable to the bioactive agent in the coating. In one embodiment, the dispersed phase comprises a polymer having a glass transition temperature ("Tg") from about 25°C to about 100°C, more narrowly from about 35°C to about 80°C, and even more narrowly from about 40°C to about 50°C. In some embodiments, the dispersed phase is amorphous but more crystalline than the matrix phase polymer. The dispersed phase has a crystallinity less than 50 weight percent (wt %), more narrowly less than 40 wt%, or even more narrowly less than 20 wt%.

The dispersed phase can include any of biocompatible polymers, as long as these polymers are immiscible or substantially immiscible with the matrix phase as defined above. In some embodiments, the dispersed phase can include polymers such as D,L polylactic acid (D,L-PLA); ply(lactide-co-glycolide) (PLGA); any derivatives, analogs, homologues, congeners, salts, copolymers, and blends thereof.

The release profile of a bioactive agent through a reservoir layer is affected by the weight to weight (wt:wt) ratio of the matrix phase polymer to the dispersed phase polymer in the reservoir layer or any other top coat layer, should a topcoat layer be used. The release profile is affected by the wt:wt ratio of the blend of matrix phase polymer and dispersed phase polymer because the bioactive agent is less permeable through the discrete phase as compared to the matrix phase. Thus, a higher weight ratio of the dispersed phase works to slow the release rate of an agent from the coating. On the other hand, to achieve a faster release rate, a higher weight ratio of matrix polymer can be used. By adjusting the ratio of polymers in a polymer blend for use as a reservoir layer or release profile controlling layer, optimal release rates may be achieved.

In one embodiment, the wt:wt ratio of matrix phase polymer to dispersed phase polymer in a reservoir layer ranges from 1:1 to 30:1, 1:1 to 25:1, 1:1 to 20:1, 1:1 to 15:1, 1:1 to 10:1, 1:1 to 8:1, 1:1 to 6:1, 1:1 to 4:1, and 1:1 to 2:1.

A single bioactive agent or a combination of bioactive agents may be contained in the coating(s), so long as the bioactive agent or a combination of agents is less permeable through the dispersed phase than through the matrix phase or impermeable through the dispersed phase. As discussed above, because the bioactive agent is less permeable or impermeable through the dispersed phase, a morphology having a dispersed phase and a matrix phase allows for slowed drug release kinetics of the agent through the coating. As used herein, the term "impermeable" refers to the permeability of by a bioactive agent described herein through the dispersed phase being about 1/5 or less, about 1/10 or less, or about 1/20 or less of the permeability of bioactive agent through the matrix phase.

In some embodiments, a coating described herein could have a release rate controlling topcoat such that the topcoat contains a matrix phase and a dispersed phase that overlays a polymer+ bioactive reservoir where the polymer is one-phase.

A preferred bioactive agent for use with the coatings of this invention is 40-O-(2-hydroxyethyl) rapamycin (everolimus).

A factor affecting the release rate profile of a bioactive agent from a coating of this invention is the weight to weight (wt:wt) ratio of bioactive agent to the total polymer content in the coating. The preferred ratio of bioactive agent to total polymer content in the coating can be from 1:1 to 1:50, 1:1 to 1:25, 1:1 to 1:20, 1:1 to 1:15, 1:1 to 1:10, 1:1 to 1:5, 1:1 to 1:3, and 1:1 to 1:2.

In some embodiments, the total polymer content refers only to the reservoir layer. In one embodiment, total polymer content refers to the reservoir layer and topcoat layer. Alternatively, it can include all polymer layers used for the coatings.

In some embodiments, the bioactive agent is everolimus, and the preferred wt:wt ratio of everolimus to total polymer of a matrix polymer/dispersed polymer blend is from about 1:1 to about 1:4, 1:1 to 1:3, or 1:1 to 1:2, depending on the release profile desired.

One of the factors affecting a release profile of coating is loading, that is the quantity of a material present in a particular coating. As used herein, loading applies to both bioactive agents and polymers. Loading is expressed as a weight of material per unit area, e.g., µg/cm². Thus, in a non-limiting example, if a 12 mm stent, which has a surface area of 0.5556 cm², is coated with a uniform layer comprising 100 µg of a bioactive agent, the loading of the bioactive agent on the stent is 179.9 µg/cm² (0.5556 cm²/100 µg). All loadings in this application are determined by a similar calculation whether bioactive agents or polymers are the subject of the loading.

For example, in one embodiment, the total loading of the bioactive agent in the coating is from about 5 to about 300 µg/cm², more narrowly from about 25 to about 200 µg/cm², and even more narrowly from about 50 to 150 µg/cm² loading.

The total loading of the blend of matrix phase polymer/dispersed phase polymer and bioactive agent in the reservoir layer also affects the release profile of the coating. In one embodiment, the total loading of the blend of matrix phase polymer/dispersed phase polymer and bioactive agent in a reservoir layer can range from about 50 to about 1000 µg/cm², preferably from about 100 to about 700 µg/cm², and presently most preferably from about 200 to about 500 µg/cm².

A coating's Tg is another factor that can control the coating's release profile. In one embodiment, the T_{g} of the coating will depend on the ratio of the matrix phase to the dispersed phase in a reservoir layer and/or a release profile controlling layer. For example, in embodiments having PEA-TEMPO as a matrix phase polymer and PGLA as a dispersed phase polymer, the Tg of the PEA-TEMPO is about 33°C and that of PLGA is about 45°C. By adjusting the blend of PEA-TEMPO and PLG, the Tg of the blend can be fine-tuned as desired. The T_{g} of a miscible blend of polymers is roughly linear with the molar ratio of the component polymers.

A further factor that can be used to affect the release rate profile of an agent from a coating is the "release profile controlling layer," or an additional layer disposed over the reservoir layer to control the release rate of the bioactive agent from the reservoir layer. In one embodiment, a release profile controlling layer comprises wt:wt ratio of a matrix phase polymer to dispersed phase polymer equivalent to that previously disclosed.

In one embodiment, the total loading of a matrix phase/dispersed phase blend on the coating in the release profile controlling layer is about 100 to 500 µg/cm², more narrowly from about 100 to about 400 µg/cm², and even more narrowly from about 100 to 450 µg/cm².

The release profile controlling layer can be used in combination with the reservoir layer to provide a controlled release of bioactive agent. In one embodiment, the wt:wt ratio of the matrix phase to dispersed phase in a reservoir layer is such that the reservoir layer has a fast release profile so that the release profile controlling layer will in fact control the actual release profile.

In some embodiments, a release profile can be one that from about 20 wt% to 60 wt% of the total amount of bioactive agent from a coating is released from the coating over about 1 to 3 days after implantation. In other embodiments, about 25 wt % to about 35 wt % of the total amount of bioactive agent from a coating is released from the coating over about a 1 day period. In another embodiment, about 30 wt % to about 50 wt % of the total amount of bioactive agent in the coating is released from the coating over about a 2 day period. In yet another embodiment, about 40 wt % to about 60 wt % of the total amount of bioactive agent in the coating is released from the coating over about a 3 day period.

In some instances, a release that is at first very rapid, i.e., essentially a "burst" may be desirable in which case the bioactive agent may be apportioned or divided between the reservoir layer and the rate-controlling layer. The bioactive agent in the release profile controlling layer will be released into the environment very shortly after exposure of the profile release controlling layer to the physiological environment; that is, in a much shorter time span even than that described above as "fast release."

A further coating construct of this invention is to first apply a primer layer to the bare surface of an implantable medical device as known to those skilled in the art. The primer layer may be applied between the reservoir layer and the surface of the implantable medical device to effect or improve adhesion of the reservoir layer to the surface of the implantable medical device. Any suitable primer layer for facilitating adhesion with the scaffolding of the stent can be used.

A method to fabricate the device comprises applying a reservoir layer directly to the bare surface of the implantable medical device or atop a previously applied primer layer and then applying a release profile controlling layer atop the reservoir layer. In this construct, the second layer may be the release profile controlling layer.

The release profile controlling layer may be directly in contact with the external environment or it may be over-coated with a topcoat layer. Generally, the topcoat layer is a thin layer intended simply to protect the underlying layers from contact with the environment until the implantable medical device has been implanted at a target location. That is, the topcoat layer does not participate in establishing a release profile. If desired, however, the topcoat layer may contain materials to improve the biocompatibility and performance of the implantable medical device. In either case, if used, a topcoat is selected that either rapidly disintegrates under physiological conditions or is sufficiently permeable as to be virtually transparent to the bioactive agent being delivered.

A neat layer of bioactive agent may be applied directly atop the primer. As used herein, "neat" means that no reservoir layer-forming or release profile controlling polymer(s) are included in the layer.

The matrix phase polymer for use as a reservoir layer can comprise PEA-TEMPO. In this matrix, D,L PLA polymer may be combined as the dispersed phase. Everolimus may be included in this reservoir coating at a loading of 50 to 100 µg/cm². In embodiments that include everolimus as the bioactive agent in a PEA-TEMPO/D,L PLA reservoir layer, the wt:wt ratio of everolimus to PEA-TEMPO/D,L PLA may be from 1:1 to 1:10, 1:1 to 1:8, 1:1 to 1.6, 1:1 to 1.4, 1:1 to 1.2 and 1:1 to 1:1.5. In one embodiment, the loading of everolimus, PEA-TEMPO, and D,L PLA in the drug reservoir layer is at about 75 to 500 µg/cm².

In one embodiment, a release profile controlling layer of a matrix phase PEA-TEMPO polymer and a dispersed phase D,L PLA polymer may be disposed over a drug reservoir layer to help control the release of active agent from the coating. In one embodiment, the wt:wt ratio of PEA-TEMPO to D,L PLA in the release profile controlling layer is as described above. The release profile controlling layer of PEA-TEMPO/D,L-PLA may have a loading of about 100 to 500 µg/cm² in the coating.

The matrix phase polymer for use as a reservoir layer can comprise PEA-TEMPO. In this matrix, a matrix phase of PLGA may be dispersed therein. In one embodiment, a drug reservoir layer comprises everolimus at a loading of 15 to 50 µg/cm²; PEA-TEMPO; and PLGA. In this embodiment, the total loading of everolimus, PEA-TEMPO, and PLGA in the reservoir layer is from about 100 to 200 µg/cm². In some embodiments, the wt:wt ratio of everlimus to PEA-TEMPO/PLGA in a reservoir layer is as described above.

In one embodiment, a release profile controlling layer may also be disposed over the reservoir layer of PEA-TEMPO, PGLA, and everolimus. In one embodiment, the release profile controlling layer comprises a blend of PEA-TEMPO and PLGA having a wt:wt ratio as described above, for example, about 10:1 to 10:9, 10:1 to 5:4, 10:1 to 10:7, 10:1 to 5:3, 10:1 to 2:1, and 10:1 to 5:2. In this embodiment, the PEA-TEMPO and PLGA blend has a total loading of about 50 to 400, more narrowly, 50 to 350, 50 to 300, 100 to 300, 100 to 200, and 120 to 160 µg/cm² in the release profile controlling layer.

The dosage of bioactive agent to be delivered will depend on factors such as, without limitation, the condition of the patient, the nature and progression of the disease or disorder, the nature of the therapy, i.e., therapeutic or prophylactic, the expected residence time of the bioactive agent at the target site (that is, its decomposition rate in vivo), the nature and type of other bioactive agents in the formulation, etc.

At present, preferred implantable medical devices for use with the coatings of this invention are stents. A stent refers generally to any device used to hold tissue in place in a patient's body. Particularly useful stents, however, are those used for the maintenance of the patency of a vessel in a patient's body when the vessel is narrowed or closed due to diseases or disorders including, without limitation, tumors (in, for example, bile ducts, the esophagus, the trachea/bronchi, etc.), benign pancreatic disease, coronary artery disease, carotid artery disease and peripheral arterial disease such as atherosclerosis, restenosis and vulnerable plaque.

Vulnerable plaque refers to a fatty build-up in an artery thought to be caused by inflammation. The vulnerable plaque is covered by a thin fibrous cap that can rupture leading to blood clot formation. A stent can be used to strengthen the wall of the vessel in the vicinity of the vulnerable plaque and act as a shield against such rupture. A stent can be used in, without limitation, neuro, carotid, coronary, pulmonary, aorta, renal, biliary, iliac, femoral and popliteal as well as other peripheral vasculatures. A stent can be used in the treatment or prevention of disorders such as, without limitation, thrombosis, restenosis, hemorrhage, vascular dissection or perforation, vascular aneurysm, chronic total occlusion, claudication, anastomotic proliferation, bile duct obstruction and ureter obstruction.

In addition to the above uses, stents may also be employed for the localized delivery of bioactive agents to specific sites in a patient's body. In some embodiments, bioactive agent delivery may be the sole purpose of the stent.

A stent used for patency maintenance is usually delivered to the target site in a compressed state and may then be expanded to fit the vessel into which it has been inserted. Once at a target location, a stent may be self-expandable or balloon expandable. In any event, due to the expansion of the stent, any coating thereon must be flexible and capable of elongation. The coatings of this invention exhibit these characteristics.

The coatings described herein may be disposed on a stent and the stent may be implanted to treat or prevent restenosis. "Treating" means that restenosis is already detected in a patient and the coated stent is implanted at the site of restenosis to retard the progress of the restenosis, that is, to slow the closure of the lumen of the vascular entity being treated. In this context, treatment also includes prophylaxis in that it may delay the onset of restenosis; that is, a coated stent is implanted in a patient before restenosis is observed in an effort to maintain the patency of the vascular entity being treated as long as possible. Treatment can also mean reducing the amount of restenosis that may form in a patient.

A bioactive agent can be any agent which is a therapeutic, prophylactic, or diagnostic agent. These agents can have anti-proliferative or anti-inflammatory properties or can have other properties such as antineoplastic, antiplatelet, anti-coagulant, anti-fibrin, antithrombotic, antimitotic, antibiotic, antiallergic, or antioxidant properties. Moreover, these agents can be cystostatic agents, agents that promote the healing of the endothelium, or agents that promote the attachment, migration and proliferation of endothelial cells while quenching smooth muscle cell proliferation. Examples of suitable therapeutic and prophylactic agents include synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules, which bind to complementary DNA to inhibit transcription, and ribozymes. Some other examples of bioactive agents include antibodies, receptor ligands, enzymes, adhesion peptides, blood clotting factors, inhibitors or clot dissolving agents, such as streptokinase and tissue plasminogen activator, antigens for immunization, hormones and growth factors, oligonucleotides such as antisense oligonucleotides and ribozymes and retroviral vectors for use in gene therapy. Examples of anti-proliferative agents include rapamycin and its functional or structural derivatives, 40-*O*-(2-hydroxy)ethyl-rapamycin (everolimus), and its functional or structural derivatives, paclitaxel and its functional and structural derivatives. Examples of rapamycin derivatives include 40-epi-(N1-tetrazolyl)-rapamycin (ABT-578), 40-*O*-(3-hydroxy)propyl-rapamycin, 40-*O*-[2-(2-hydroxy)ethoxy]ethyl-rapamycin, and 40-*O*-tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin^{®} from Pharmacia & Upjohn, Peapack N.J.), and mitomycin (e.g. Mutamycin^{®} from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as Angiomax^{®} (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), super oxide dismutases, super oxide dismutase mimetic, 4-amino-2,2,6,6-tetramethylpiperidine-1-oxyl (4-amino-TEMPO), estradiol, anticancer agents, dietary supplements such as various vitamins, and a combination thereof. Examples of anti-inflammatory agents including steroidal and non-steroidal anti-inflammatory agents include biolimus, tacrolimus, dexamethasone, clobetasol, corticosteroids or combinations thereof. Examples of such cytostatic substances include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Prinivil^{®} and Prinzide^{®} from Merck & Co., Inc., Whitehouse Station, NJ). An example of an antiallergic agent is permirolast potassium. Other therapeutic substances or agents which may be appropriate include alpha-interferon, pimecrolimus, imatinib mesylate, midostaurin, and genetically engineered epithelial cells. The foregoing substances can also be used in the form of prodrugs or co-drugs thereof. The foregoing substances also include metabolites thereof and/or prodrugs of the metabolites. The foregoing substances are listed by way of example and are not meant to be limiting. Other active agents which are currently available or that may be developed in the future are equally applicable.

Any implantable medical device can be coated with polymer blends of this invention. As used herein an implantable medical device refers to any type of appliance that is totally or partly introduced, surgically or medically, into a patient's body or by medical intervention into a natural orifice. As used herein, a patient refers to purposes for human as well as veterinary purposes. Examples of implantable medical devices include, without limitation, implantable cardiac pacemakers and defibrillators; leads and electrodes for the preceding; implantable organ stimulators such as nerve, bladder, sphincter and diaphragm stimulators; cochlear implants; prostheses, self-expandable stents, balloon-expandable stents, stent-grafts, and grafts; artificial heart valves; and cerebrospinal fluid shunts.

Implantable medical devices constructed of virtually any biocompatible material, as such are presently known or as such may be developed in the future, may be used with a coating of this invention. For example, without limitation, an implantable medical device useful with a coating of this invention may be made of one or more biocompatible metals or alloys thereof including, but not limited to, cobalt-chromium alloy (ELGILOY, L-605), cobalt-nickel alloy (MP-35N), 316L stainless steel, high nitrogen stainless steel, e.g., BIODUR 108, nickel-titanium alloy (NITINOL), tantalum, platinum, platinum-iridium alloy, gold and combinations thereof.

An implantable medical device suitable for use with the coatings herein may also be made of polymers that are biocompatible and biostable or biodegradable, the latter term including bioabsorbable and/or bioerodable. Among useful biocompatible, relatively biostable polymers are, without limitation polyacrylates, polymethacryates, polyureas, polyurethanes, polyolefins, polyvinylhalides, polyvinylidenehalides, polyvinylethers, polyvinylaromatics, polyvinylesters, polyacrylonitriles, alkyd resins, polysiloxanes and epoxy resins.

Biocompatible, biodegradable polymers include naturally-occurring polymers such as, without limitation, collagen, chitosan, alginate, fibrin, fibrinogen, cellulosics, starches, dextran, dextrin, hyaluronic acid, heparin, glycosaminoglycans, polysaccharides and elastin.

One or more synthetic or semi-synthetic biocompatible, biodegradable polymers may also be used to fabricate an implantable medical device useful with this invention. A synthetic polymer refers to one that is created wholly in the laboratory while a semi-synthetic polymer refers to a naturally-occurring polymer than has been chemically modified in the laboratory. Examples of synthetic polymers include, without limitation, polyphosphazines, polyphosphoesters, polyphosphoester urethane, polyhydroxyacids, polyhydroxyalkanoates, polyanhydrides, polyesters, polyorthoesters, polyamino acids, polyoxymethylenes, poly(ester-amides) and polyimides.

Blends and copolymers of the above polymers may also be used and are within the scope of this invention.

### EXAMPLES

The examples presented in this section are provided by way of illustration. Each of the examples that follow relates to the coating of a 12 mm stent having a surface area of 0.5556 cm².

### EXAMPLE 1: Constructs having a blend of PEA-TEMPO/D,L-PLA on a 12 mm stent. Example 1a

Stents were made using everolimus at a loading of 100 µg/cm² in the coating. Everolimus was included in a drug reservoir layer of PEA-TEMPO, having a drug-to-polymer ratio of 1:1 and a total loading of 112 µg/cm². Agent release was further controlled by adding a release profile controlling layer having a blend of PEA-TEMPO and D,L PLA. The ratio of polymer-to-polymer of the release profile controlling layer was held constant at 1:1 with a total loading of 220 µg/cm².

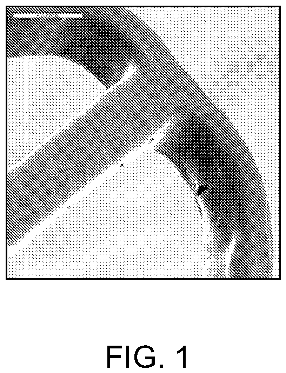

Figure 1 illustrates an SEM image of a portion of a stent formed according to Example 1a after simulated use testing. As depicted, the coating integrity after simulated use testing was not disrupted.

### Example 1b

Stents were made using everolimus at a loading of 50 µg/cm² in the coating. Everolimus was included in a first, drug reservoir layer of PEA-TEMPO, having a drug-to-polymer ratio of 1:1 and a total loading of 56 µg/cm². Agent release was further controlled by adding a release profile controlling layer of PEA-TEMPO as a matrix phase polymer and D,L PLA as a dispersed phase polymer. The ratio of polymer-to-polymer in the rate controlling layer was held constant at 1:1 with a total loading of 240 µg/cm².

### Example 1c

Stents were made using everolimus at a loading of 100 µg/cm² in a coating. Everolimus was included in a first drug reservoir layer of a blend of PEA-TEMPO as the matrix phase polymer and D,L PLA as the dispersed phase polymer, having a drug-to-polymer ratio of 1:2 and a total loading in the reservoir layer of 168 µg/cm². The ratio of polymer-to-polymer of the PEA-TEMPO and D,L-PLA blend was 1:1.5. Agent release was further controlled by adding a release profile controlling layer having a blend of PEA-TEMPO as a matrix phase polymer and D,L PLA as a dispersed phase polymer. The ratio of polymer-to-polymer of release profile controlling layer was held constant at 1:1.5 with a total loading of 112 µg/cm².

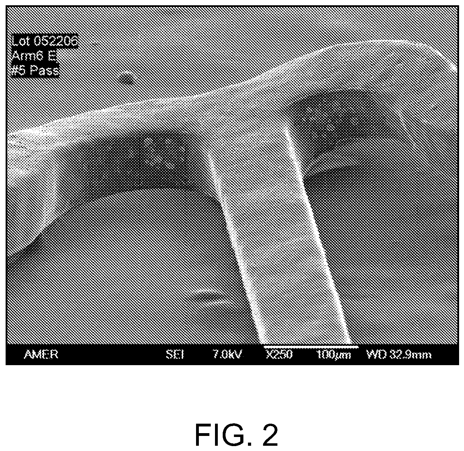

Figure 2 is a SEM image depicting a portion of a stent formed according to Example 1c after simulated use testing. As depicted, the coating integrity of the stent after simulated use testing was not disrupted.

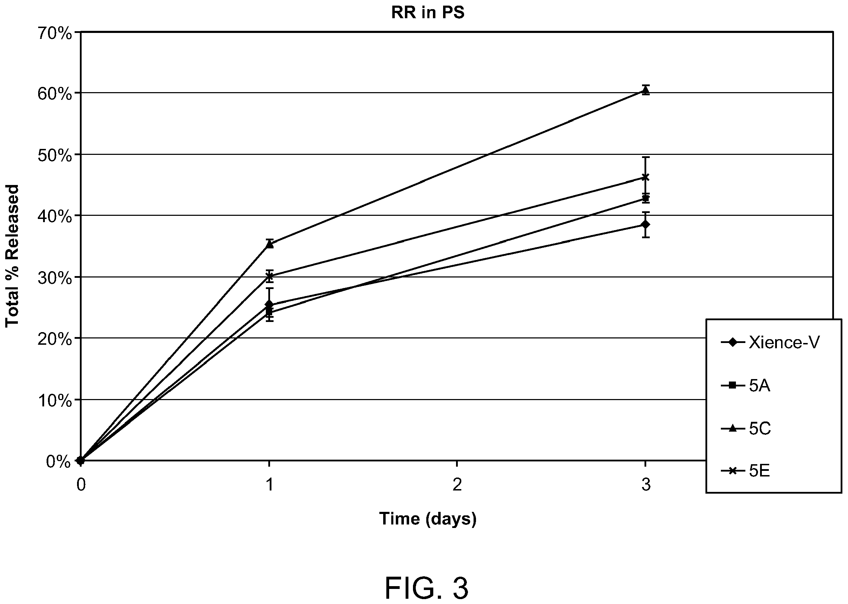

Figure 3 is a graph of the cumulative percent everolimus released into porcine serum (1 day and 3 day) for each of the Example 1a, 1b, and 1c as compared to a everolimus/poly(vinylidene fluoride-co-hexafluoropropene) coating. As depicted by Figure 1, Example 1a most closely matches the everolimus/poly(vinylidene fluoride-co-hexafluoropropene) coating release rate, and the coating integrities for Examples 1a and 1b were identical after simulated use testing.

### EXAMPLE 2: Constructs having a blend of PEA-TEMPO/PLGA on a 12 mm stent.

Stents were made using everolimus at a loading of 25 µg/cm² in the coating. Everolimus was included in a drug reservoir layer of a blend of PEA-TEMPO and PGLA, having a drug-to-polymer ratio of 1:10 and a total loading of 154 µg/cm². The ratio of polymer-to-polymer of the PEA-TEMPO and PGLA blend was 4:3. Agent release was further controlled by adding a second layer of a PEA-TEMPO and PLGA blend. The ratio of polymer-to-polymer of the release profile controlling layer was held constant at 4:3 with a total loading of 140 µg/cm².

## Claims

1. A coating for an implantable medical device comprising a drug reservoir layer that comprises:
a major matrix phase comprising at least one matrix phase polymer having a glass
transition temperature ("Tg") of -10°C to 50°C, the at least one matrix phase polymer being a poly(ester amide) polymer comprising poly {[N,N'-sebacoyl-bis-(L-leucine)-1,6-hexylene diester]-co-[N,N'-sebacoyl-L-lysine 4-amino-2,2,6,6-tetramethylpiperidine-N-oxide] (PEA-TEMPO);
a minor dispersed phase comprising at least one dispersed phase polymer; and
the drug reservoir layer further comprises a bioactive agent which is everolimus;
wherein less than 10% of the dispersed phase is miscible in the matrix phase,
wherein the bioactive agent is less permeable through the dispersed phase than the matrix phase, and
wherein the coating has a thickness less than about 5 microns.

2. The coating according to Claim 1, wherein the implantable medical device is a stent.

3. The coating according to Claim 1, wherein the dispersed phase polymer is D,L-polylactide (D,L-PLA) or poly(lactide-co-glycolide) (PLGA), and wherein the bioactive agent, matrix phase polymer and dispersed phase polymer form a reservoir layer blend;
wherein when the reservoir layer blend is everolimus, PEA-TEMPO/D,L PLA, then the reservoir layer blend has a loading of everolimus of 50 to 100 µg/cm², and the total loading of everolimus, PEA-TEMPO/D,L PLA in the drug reservoir layer is from about 75 to 500 µg/cm²; and
when the reservoir layer blend is everolimus, PEA-TEMPO/PLGA, then the reservoir layer blend has a loading of everolimus of 15 to 50 µg/cm², and the total loading of everolimus, PEA-TEMPO/PLGA in the drug reservoir layer is from about 100 to 200 µg/cm².

4. The coating according to Claim 1, which is capable of complete degradation in a period of less than about 6 months in a physiological environment.

5. The coating according to Claim 1, wherein about 25 wt % to about 35 wt % of the bioactive agent is released from the coating over about a 1 day period.

6. The coating according to Claim 1, wherein about 30 wt % to about 50 wt % of the bioactive agent is released from the coating over about a 2 day period.

7. The coating according to Claim 1, wherein about 40 wt % to about 60 wt % of the bioactive agent is released from the coating over about a 3 day period.

8. The coating according to Claim 1, wherein the coating further comprises a release profile controlling layer disposed over the drug reservoir layer.

9. The coating according to Claim 1 or 8, wherein the dispersed phase polymer is selected from the group consisting of D,L-PLA, poly(lactide-co-glycolide) (PLGA), and blends thereof.

10. The coating according to Claim 8, wherein the release profile controlling layer comprises a blend of a matrix phase polymer and a dispersed phase polymer, and (a) PEA-TEMPO is the matrix phase polymer and (b) the dispersed phase polymer is selected from the group consisting of: D,L-PLA, PLGA, and combinations thereof, and
wherein when the blend is PEA-TEMPO/D,L PLA, then the blend has a loading of about 100 to 500 µg/cm² in the release profile controlling layer;
when the blend is PEA-TEMPO/PLGA, then the blend has a loading of about 50 to 400 µg/cm² in the release profile controlling layer; and
when the blend is PEA-TEMPO and a combination of D,L PLA/PLGA, then the blend has a loading of about 100 to 250 µg/cm² in the release profile controlling layer.

11. The coating according to Claim 8, wherein about 25 wt % to about 35 wt % of the bioactive agent is released from the coating over about a 1 day period.

12. The coating according to Claim 8, wherein about 30 wt % to about 50 wt % of the bioactive agent is released from the coating over about a 2 day period.

13. The coating according to Claim 8, wherein about 40 wt % to about 60 wt % of the bioactive agent is released from the coating over about a 3 day period.

14. The coating according to Claim 1 or 8, wherein the release profile controlling layer comprises a blend of PEA-TEMPO and PLGA, and the wt:wt ratio of the PEA-TEMPO polymer to the PLGA polymer in the blend is 10:1 to 10:9 .

15. The coating according to claim 8, wherein the total loading of the bioactive agent in the drug reservoir layer is from 50 to 100 µg/cm², and the dispersed phase polymer is selected from the group consisting of: D,L-PLA, PLGA, and combinations thereof,
wherein the total loading of everolimus, PEA-TEMPO, and the dispersed phase polymer in the reservoir layer is about 100 to about 700 µg/cm².

16. The coating according to Claim 3, wherein the wt:wt ratio of everolimus to the sum of PEA-TEMPO and the D,L-PLA dispersed phase polymer in the drug reservoir layer is about 1:1 to about 1:10.

17. The coating according to Claim 10,
wherein the blend in the release profile controlling layer has a weight ratio of (a) to (b) of about 4:3; and
wherein the blend has a total loading of (a) and (b) of about 120 to 160 µg/cm².

18. The coating according to Claim 1 or 8, wherein the matrix phase polymer and the dispersed phase polymer is a single polymer comprising immiscible polymeric blocks, forming the matrix phase and the dispersed phase.

19. A stent having a coating as defined in any one of the preceding claims 1-18, for use in the treatment or prevention of a medical condition.

## Patentansprüche

1. Eine Beschichtung für ein implantierbares medizinisches Gerät umfassend eine Arzneimittelreservoirschicht, die folgendes umfasst:
eine Hauptmatrixphase umfassend mindestens ein Matrixphasenpolymer, das eine
Glasübergangstemperatur ("Tg") von -10°C bis 50°C hat, wobei das mindestens eine Matrixphasenpolymer ein Polyesteramid-Polymer umfassend Poly{[N,N'-sebacoyl-bis-(L-leucin)-1,6-hexylendiester]-co-[N,N'-sebacoyl-L-lysin 4-amino-2,2,6,6-tetramethylpiperidin-N-oxid] (PEA-TEMPO) ist;
eine dispergierte Nebenphase umfassend mindestens ein Polymer der dispergierten Phase; und
die Arzneimittelreservoirschicht weiterhin ein bioaktives Mittel umfasst, das Everolimus ist;
wobei weniger als 10% der dispergierten Phase in der Matrixphase mischbar ist,
wobei das bioaktive Mittel weniger permeabel durch die dispergierte Phase als durch die Matrixphase ist, und
wobei die Beschichtung eine Dicke von weniger als ca. 5 Mikrometer hat.

2. Die Beschichtung nach Anspruch 0, wobei das implantierbare medizinische Gerät ein Stent ist.

3. Die Beschichtung nach Anspruch 1, wobei das Polymer der dispergierten Phase D,L-Polylactid (D,L-PLA) oder Poly(lactid-co-glycolid) (PLGA) ist, und wobei das bioaktive Mittel, das Matrixphasenpolymer und das Polymer der dispergierten Phase eine Reservoirschichtmischung bilden;
wobei, wenn die Reservoirschichtmischung Everolimus, PEA-TEMPO/D,L PLA ist, die Reservoirschichtmischung eine Menge an Everolimus von 50 bis 100 µg/cm² hat, und die Gesamtmenge von Everolimus, PEA-TEMPO/D,L PLA in der Arzneimittelreservoirschicht von ca. 75 bis 500 µg/cm² reicht; und
wobei, wenn die Reservoirschichtmischung Everolimus, PEA-TEMPO/PLGA ist, die Reservoirschichtmischung eine Menge an Everolimus von 15 bis 50 µg/cm² hat, und die Gesamtmenge von Everolimus, PEA-TEMPO/PLGA in der Arzneimittelreservoirschicht von ca. 100 bis 200 µg/cm² ist.

4. Die Beschichtung nach Anspruch 1, welche in einem Zeitraum von weniger als ca. 6 Monaten in einer physiologischen Umgebung vollständig abgebaut werden kann.

5. Die Beschichtung nach Anspruch 1, wobei ca. 25 Gew.-% bis ca. 35 Gew.-% des bioaktiven Mittels aus der Beschichtung über einen Zeitraum von ca. 1 Tag freigesetzt wird.

6. Die Beschichtung nach Anspruch 1, wobei ca. 30 Gew.-% bis ca. 50 Gew.-% des bioaktiven Mittels aus der Beschichtung über einen Zeitraum von ca. 2 Tagen freigesetzt wird.

7. Die Beschichtung nach Anspruch 1, wobei ca. 40 Gew.-% bis ca. 60 Gew.-% des bioaktiven Mittels aus der Beschichtung über einen Zeitraum von ca. 3 Tagen freigesetzt wird.

8. Die Beschichtung nach Anspruch 1, wobei die Beschichtung weiterhin eine Schicht zur Freisetzungsprofilkontrolle über der Arzneimittelreservoirschicht umfasst.

9. Die Beschichtung nach Anspruch 1 oder 8, wobei das Polymer der dispergierten Phase ausgewählt aus der Gruppe bestehend aus D,L-PLA, Poly(lactid-co-glycolid) (PLGA), und Mischungen davon ist.

10. Die Beschichtung nach Anspruch 8, wobei die Schicht zur Freisetzungsprofilkontrolle eine Mischung aus einem Matrixphasenpolymer und einem Polymer der dispergierten Phase umfasst, und (a) PEA-TEMPO das Matrixphasenpolymer ist und (b) das Polymer der dispergierten Phase ausgewählt aus der Gruppe bestehend aus D,L-PLA, PLGA, und Kombinationen davon ist, und
wobei, wenn die Mischung PEA-TEMPO/D,L PLA ist, die Mischung eine Menge von ca. 100 bis 500 µg/cm² in der Schicht zur Freisetzungsprofilkontrolle hat;
wenn die Mischung PEA-TEMPO/PLGA ist, die Mischung eine Menge von ca. 50 bis 400 µg/cm² in der Schicht zur Freisetzungsprofilkontrolle hat; und
wenn die Mischung PEA-TEMPO und eine Kombination aus D,L-PLA/PLGA ist, die Mischung eine Menge von ca. 100 bis 250 µg/cm² in der Schicht zur Freisetzungsprofilkontrolle hat.

11. Die Beschichtung nach Anspruch 8, wobei ca. 25 Gew.-% bis ca. 35 Gew.-% des bioaktiven Mittels aus der Beschichtung über einen Zeitraum von ca. 1 Tag freigesetzt wird.

12. Die Beschichtung nach Anspruch 8, wobei ca. 30 Gew.-% bis ca. 50 Gew.-% des bioaktiven Mittels aus der Beschichtung über einen Zeitraum von ca. 2 Tagen freigesetzt wird.

13. Die Beschichtung nach Anspruch 8, wobei ca. 40 Gew.-% bis ca. 60 Gew.-% des bioaktiven Mittels aus der Beschichtung über einen Zeitraum von ca. 3 Tagen freigesetzt wird.

14. Die Beschichtung nach Anspruch 1 oder 8, wobei die Schicht zur Freisetzungsprofilkontrolle eine Mischung aus PEA-TEMPO und PLGA umfasst, und wobei das Verhältnis (Gewicht:Gewicht) vom PEA-TEMPO-Polymer zum PLGA-Polymer in der Mischung von 10:1 bis 10:9 reicht.

15. Die Beschichtung nach Anspruch 8, wobei die Gesamtmenge des bioaktiven Mittels in der Arzneimittelreservoirschicht von 50 bis 100 µg/cm² reicht, und das Polymer der dispergierten Phase ausgewählt aus der Gruppe bestehend aus: D,L-PLA, PLGA, und Kombinationen davon ist,
wobei die Gesamtmenge von Everolimus, PEA-TEMPO, und das Polymer der dispergierten Phase in der Reservoirschicht von ca. 100 bis ca. 700 µg/cm² ist.

16. Die Beschichtung nach Anspruch 3, wobei das Verhältnis (Gewicht:Gewicht) von Everolimus zu der Summe von PEA-TEMPO und dem D,L-PLA-Polymer der dispergierten Phase in der Arzneimittelreservoirschicht von ca. 1:1 bis ca. 1:10 reicht.

17. Die Beschichtung nach Anspruch 10,
wobei die Mischung in der Schicht zur Freisetzungsprofilkontrolle ein Gewichtsverhältnis von (a) bis (b) von ca. 4:3 hat; und
wobei die Mischung eine Gesamtmenge von (a) und (b) von ca. 120 bis 160 µg/cm² hat.

18. Die Beschichtung nach Anspruch 1 oder 8, wobei das Matrixphasenpolymer und das Polymer der dispergierten Phase ein einziges Polymer umfassend unmischbare polymere Blöcke ist, welche die Matrixphase und die dispergierte Phase ausmachen.

19. Ein Stent, der eine Beschichtung wie in einem der vorhergehenden Ansprüche 1-18 definiert hat, zur Verwendung in der Behandlung oder Prävention von einem medizinischen Zustand.

## Revendications

1. Un revêtement pour un dispositif médical implantable comprenant une couche de réservoir de médicament qui comprend:
une phase de matrice majeure comprenant au moins un polymère de phase de matrice ayant une température de transition vitreuse ("Tg") de -10 °C à 50 °C, l'au moins un polymère de phase de matrice étant un polymère de poly(ester amide) comprenant du poly{[N,N'-sébacoyl-bis-(L-leucine)-1,6-hexylène diester]-co-[N,N'-sébacoyl-L-lysine 4-amino-2,2,6,6-tétraméthylpiperidine-N-oxyde] (PEA-TEMPO);
une phase dispersée mineure comprenant au moins un polymère de phase dispersée; et
la couche de réservoir de médicament comprend en outre un agent bioactif qui est l'évérolimus;
dans lequel moins du 10 % de la phase dispersée est miscible dans la phase de matrice,
dans lequel l'agent bioactif est moins perméable à travers la phase dispersée que à travers la phase de matrice, et
dans lequel le revêtement a une épaisseur inférieure à environ 5 micromètres.

2. Le revêtement selon la revendication 0, dans lequel le dispositif médical implantable est un stent.

3. Le revêtement selon la revendication 1, dans lequel le polymère de phase dispersée est le D,L-polylactide (D,L-PLA) ou le poly(lactide-co-glycolide) (PLGA), et dans lequel l'agent bioactif, le polymère de phase de matrice et le polymère de phase dispersée forment un mélange de couche de réservoir;
dans lequel, lorsque le mélange de la couche de réservoir est évérolimus, PEA-TEMPO/D,L PLA, alors le mélange de couche de réservoir a une charge totale d'évérolimus de 50 à 100 µg/cm², et la charge totale d'évérolimus, PEA-TEMPO/D,L PLA dans la couche de réservoir de médicament est d'environ 75 à 500 µg/cm² ; et
lorsque le mélange de la couche de réservoir est évérolimus, PEA-TEMPO/PLGA, alors le mélange de la couche de réservoir a une charge d'évérolimus de 15 à 50 µg/cm², et la charge totale d'évérolimus, PEA-TEMPO/PLGA dans la couche de réservoir de médicament est d'environ 100 à 200 µg/cm².

4. Le revêtement selon la revendication 1, qui peut être complètement dégradé dans une période de moins d'environ 6 mois dans un environnement physiologique.

5. Le revêtement selon la revendication 1, dans lequel d'environ 25 % en poids à environ 35 % en poids de l'agent bioactif est libéré du revêtement au cours d'environ 1 jour.

6. Le revêtement selon la revendication 1, dans lequel d'environ 30 % en poids à environ 50 % en poids de l'agent bioactif est libéré du revêtement au cours d'environ 2 jours.

7. Le revêtement selon la revendication 1, dans lequel d'environ 40 % en poids à environ 60 % en poids de l'agent bioactif est libéré du revêtement au cours d'environ 3 jours.

8. Le revêtement selon la revendication 1, dans lequel le revêtement comprend en outre une couche de contrôle du profil de libération disposée au-dessus de la couche de réservoir de médicament.

9. Le revêtement selon la revendication 1 ou 8, dans lequel le polymère de la phase dispersée est choisi dans le groupe constitué de D,L-PLA, poly(lactide-co-glycolide) (PLGA), et des mélanges de ceux-ci.

10. Le revêtement de la revendication 8, dans lequel la couche de contrôle du profil de libération comprend un mélange d'un polymère de phase de matrice et un polymère de phase dispersée, et (a) PEA-TEMPO est le polymère de la phase de matrice et (b) le polymère de la phase dispersée est choisi dans le groupe constitué de : D,L-PLA, PLGA, et des combinaisons de ceux-ci, et
dans lequel, lorsque le mélange est PEA-TEMPO/D,L PLA, alors le mélange a une charge d'environ 100 à 500 µg/cm² dans la couche de contrôle du profil de libération;
lorsque le mélange est PEA-TEMPO/PLGA, alors le mélange a une charge d'environ 50 à 400 µg/cm² dans la couche de contrôle du profil de libération; et
lorsque le mélange est PEA-TEMPO et une combinaison de D,L PLA/PLGA, alors le mélange a une charge d'environ 100 à 250 µg/cm² dans la couche de contrôle du profil de libération.

11. Le revêtement selon la revendication 8, dans lequel d'environ 25 % en poids à environ 35 % en poids de l'agent bioactif est libéré du revêtement au cours d'environ 1 jour.

12. Le revêtement selon la revendication 8, dans lequel d'environ 30 % en poids à environ 50 % en poids de l'agent bioactif est libéré du revêtement au cours d'environ 2 jours.

13. Le revêtement selon la revendication 8, dans lequel d'environ 40 % en poids à environ 60 % en poids de l'agent bioactif est libéré du revêtement au cours d'environ 3 jours.

14. Le revêtement selon la revendication 1 ou 8, dans lequel la couche de contrôle du profil de libération comprend un mélange de PEA-TEMPO et PLGA, et le rapport poids:poids du polymère PEA-TEMPO au polymère PLGA dans le mélange est de 10:1 à 10:9.

15. Le revêtement selon la revendication 8, dans lequel la charge totale de l'agent bioactif dans la couche de réservoir de médicament est de 50 à 100 µg/cm², et le polymère de la phase dispersée est choisi dans le groupe constitué de: D,L-PLA, PLGA, et des combinaisons de ceux-ci,
dans lequel la charge totale d'évérolimus, PEA-TEMPO, et le polymère de la phase dispersée dans la couche de réservoir est d'environ 100 à environ 700 µg/cm².

16. Le revêtement selon la revendication 3, dans lequel le rapport poids:poids d'évérolimus à la somme de PEA-TEMPO et de polymère de la phase dispersée D,L-PLA dans la couche de réservoir de médicament est d'environ 1:1 à environ 1:10.

17. Le revêtement selon la revendication 10,
dans lequel le mélange dans la couche de contrôle du profil de libération a un rapport en poids de (a) à (b) d'environ 4:3, et
dans lequel le mélange a une charge totale de (a) et (b) d'environ 120 à 160 µg/cm².

18. Le revêtement selon la revendication 1 ou 8, dans lequel le polymère de la phase de matrice et le polymère de la phase dispersée est un seul polymère comprenant des blocs polymères non miscibles, formant la phase de matrice et la phase dispersée.

19. Un stent ayant un revêtement tel que défini dans l'une quelconque des revendications précédentes 1-18, pour l'utilisation dans le traitement ou la prévention d'une condition médicale.
